# EUROPEAN PATENT SPECIFICATION

(11) **EP 1 435 999 B1**
(45) Date of publication and mention of the grant of the patent: **18.07.2012**
(21) Application number: 01988010.3
(22) Date of filing: 29.11.2001
(51) Int. Cl.: A61K 38/34, A61P 29/00

(54) **COMBINATION OF TWO BIOACTIVE REGIONS OF PRO-OPIOMELANOCORTIN HORMONE**
KOMBINATION VON ZWEI BIOAKTIVEN REGIONEN DES PRO-OPIOMELANOCORTINS
COMBINAISON DE DEUX REGIONS BIOACTIVES DE L'HORMONE PRO-OPIOMELANOCORTINE

(30) Priority: 18.10.2001 BA 010898
(43) Date of publication of application: 14.07.2004
(73) Proprietor: Ademovic, Zlatko, 76290 Odzak (BA); Mikus, Darko, 21 000 Split (HR)
(72) Inventor: Ademovic, Zlatko, 76290 Odzak (BA); Stambuk, Nikola, 10 000 Zagreb (HR); Konjevoda, Pasko, 10 000 Zagreb (HR); Mikus, Darko, 21 000 Split (HR)
(74) Representative: Ros, Zlata
(86) International application number: PCT/BA2001/000005
(87) International publication number: WO 2003/033017

(56) References cited:
- P. KONJEVODA ET AL.: "Cytoprotective effects of met-enkephalin and alpha-MSH on ethanol induced gastric lesions in rats." JOURNAL OF PHYSIOLOGY-PARIS, vol. 95, no. 1-6, January 2001 (2001-01) - December 2001 (2001-12), pages 277-281, XP002232277 cited in the application
- J. M. LIPTON ET AL.: "Anti-inflammatory actions of the neuroimmunomodulator alpha-MSH" IMMUNOLOGY TODAY, ELSEVIER PUBLICATIONS, CAMBRIDGE, GB, vol. 18, no. 3, March 1997 (1997-03), pages 140-145, XP002902200 ISSN: 0167-5699 cited in the application
- N.P. PLOTNIKOFF ET AL.: "Methionine enkephalin: a new cytokine - Human studies." CLINICAL IMMUNOLOGY AND IMMUNOPATHOLOGY, vol. 82, no. 2, February 1997 (1997-02), pages 93-101, XP002232278

### SEQUENCE LISTING

<110> Ademovic, Zlatko et al.
<120> Additive Cytoprotective Effects of Two Bioactive Regions of Pro-opiomelanocortin Hormone
<140> PCT/BA 01/00005
   <141> 2001-11-29
<160> 5
<170> PatentIn version 3.1
<210> 1
   <211> 13
   <212> PRT
   <213> Artificial Sequence
<220>
   <223> Artificial amino acid sequence with a gap, numbered as a plurality (combination) of two separate sequences specified with separate sequence identifiers 1 and 2 (or vice versa). The combination (plurality) of sequence identifiers 1 and 2 enables better and stronger cytoprotective and pharmacologic effects than the ones obtained by means of the individual amino acid sequence identifiers.
<400> 1
<210> 2
   <211> 5
   <212> PRT
   <213> Artificial Sequence
<220>
<223> Artificial amino acid sequence with a gap, numbered as a plurality (combination) of two separate sequences specified with separate sequence identifiers 1 and 2 (or vice versa). The combination (plurality) of sequence identifiers 1 and 2 enables better and stronger cytoprotective and pharmacologic effects than the ones obtained by means of the individual amino acid sequence identifiers.
<400> 2
   Tyr Gly Gly Phe Met
   1 5
<210> 3
   <211> 23
   <212> PRT
   <213> Artificial Sequence
<220>
<223> Designed amino acid sequence that is made up of two non-contiguous segments of a larger sequence or of segments from different sequences numbered as a separate sequence, with a separate sequence identifier (SEQ ID NO: 3).
<400> 3
   Tyr Gly Gly Phe Met Ser Tyr Ser Met Glu His Phe Arg Trp Gly Lys
   1 5 10 15
   Pro Val Tyr Gly Gly Phe Met
   20
<210> 4
   <211> 18
   <212> PRT
   <213> Artificial Sequence
<220>
<223> Designed amino acid sequence that is made up of two non-contiguous segments of a larger sequence or of segments from different sequences numbered as a separate sequence, with a separate sequence identifier (SEQ ID NO: 4).
<400> 4
   Tyr Gly Gly Phe Met Ser Tyr Ser Met Glu His Phe Arg Trp Gly Lys
   1 5 10 15
   Pro Val
<210> 5
   <211> 18
   <212> PRT
   <213> Artificial Sequence
<220>
<223> Designed amino acid sequence that is made up of two non-contiguous segments of a larger sequence or of segments from different sequences numbered as a separate sequences, with a separate sequence identifier (SEQ ID NO: 5).
<400> 5

## Description

### 1) TECHNICAL AREA OF THE INVENTION

The invention is a combination of two bioactive peptides extracted from the pro-opiomelanocortin hormone, that enables additive cytoprotective effects and modulation of the inflammatory response and tissue/wound healing. The combination of two pro-opiomelanocortin of peptides enables better pharmacologic effects and tissue leasion healing.

J.M. Lipton et al. (Immunol. Today 18 (3) (1997) 140-145) discloses the antiinflammatory actions of the neuroimmunomodulator α-MSH. N.P. Plotnikoff et al. (Clin. Immuno. Immunop. 82 (2) (1997) 93-101) discloses methionine enkephalin (met-enkaphalin) and its effects on human immune function.

### 2) TECHNICAL PROBLEM

Bioactive parts of the protein and gene sequences are often 5 to 15 amino acid long and repetitive peptides, separated by the larger amino acid blocks of undefined function (1-5). Computational analyses may be used for the extraction of such motifs and subsequent. manipulation with the bioactive effects their protein regions (1-5). The invention represents a combination of two repetitive and bioactive regions of the pro-opiomelanocortin hormone that exhibits additive cytoprotective and pharmacological effects.

### 3) TECHNICAL DESCRIPTION

Repetitive peptide motifs within a single protein or a protein family represent, together with their matching complementary sequences, regions that are lined to the bioactivity of the larger molecular structures (1-5).

Modern programing techniques, developement of the softwares and databases of the protein and gene structures enabled the computer modelling of repetitive, bioactive and complementary structures of the large number of different proteins (1-5). Consequently, the extraction of short and repetitive motifs may be done on a personal computer quickly and easily (1-5). We analyse repetitive and bioactive peptides of the pro-opiomelanocortin molecule by means of the software SCAN (3-5) in order to obtain possible combination of elements with additive pharmacologic and cytoprotective effects.

### 4) PURPOSE OF THE INVENTION (Description of the invention)

The purpose of the invention is to obtain additive cytoprotective pharmacologic effects by combining two repetitive and bioactive peptides of the pro-opiomelanocortin hormone molecule.

The inovation is in the fact that, instead of the random pharmacologic screening of different pro-opiomelanocortin protein motifs, software SCAN was used to extract two repetitive and bioactive sequences (SYSMEHFRWGKPV and YGGFM). Two extracted sequences are present in the following molecules: pro-opiomelanocortin precursor, pro-opiomelanocortin, corticotropin, melanotropin, lipotropin beta, proenkephalin, preproenkephalin, endorphin beta and adrenorphin (Table 1).

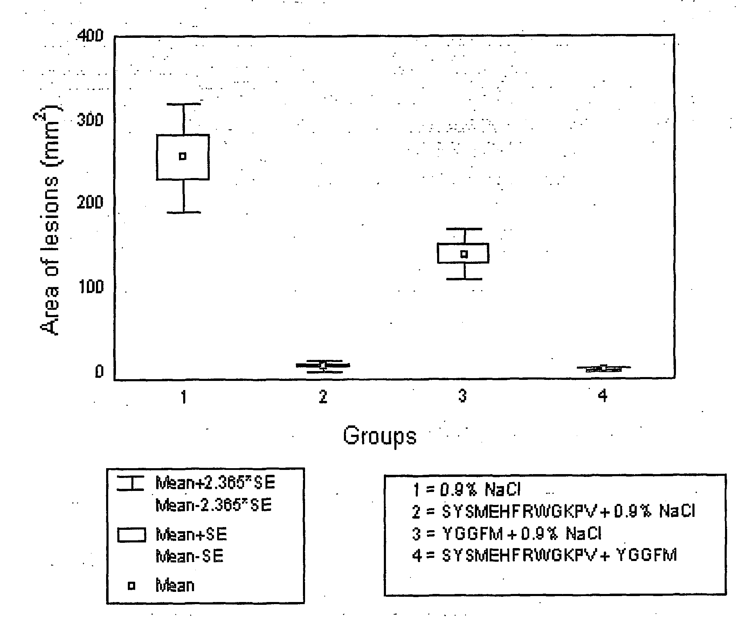

Bioactive peptide fragments SYSMEHFRWGKPV and YGGFM were tested individually and in combination, by means of the standard cytoprotection model of the 96% ethanol induced gastric lesions in male Wistar rats (6, 7). In the control group of 8 animals treated with physiological saline the area of gastric lesions was 255.5±78.1 mm² (mean ± standard deviation, Figure 1) (7). In the group of 8 animals treated with the combination of peptides SYSMEHFRWGKPV (1 mg/kg) and YGGFM (10 mg/kg) the area of gastric lesions was 0.3±0.7 mm² (p < 0.05 compared to controls, Figure 1) (7). In the group of 8 animals treated with peptide SYSMEHFRWGKPV (1 mg/kg) the area of gastric lesions was 5.9 +8 mm² (p < 0.05 compared to controls, Figure 1) (7). In the group of 8 animals treated with peptide YGGFM (10 mg/kg) the area of gastric lesions was 139.4±36.1 mm² (p < 0.05 compared to control, Figure 1) (7). The combination of peptides SYSMEHFRWGKPV and YGGFM that provided best cytoprotective effect may be used in medicine as a medicament. Peptides or their combination did not show any iritative effect on the normal gastric mucosa (7).

### 5) FIGURES AND TABLES

Table 1 defines the combination of two bioactive sequences of pro-opiomelanocortin, and three combinations of their chemical structural formulas. Figure 1 defines the cytoprotective effects of the bioactive peptide fragments SYSMEHFRWGKPV and YGGFM of the pro-opiomelanocortin molecule, and their combination, in the model of the ethanol induced gastric lesions in rats.

### 6) DESCRIPTION OF THE POSSIBLE APPLICATIONS OF THE INVENTION

Bioactive sequences, and the combination of their structural formulas defined in Table 1, may be used as the bioactive peptides for the modulation of the inflammation and wound healing. The purpose of defining structural formulas of the invention, from the bioactive regions of the pro-opiomelanocortin hormone (6-9), is to obtain structural formulas (sequences) of the potential drugs in a quick and efficient way.

The aim of the invention is to obtain the drug that prevents and stops tissue and organ damage in inflammatory and autoimmune diseases, as well as in trauma, infection and burns of the:
1. connective tissue, joints and bones
2. central and peripheral nervous system, optic nerve, eye and ear
3. skin, hair and nails
4. digestive system, liver, pancreas, oral cavity, teeth and sinuses
5. immune system, bone marrow lymph nodes and spleen
6. cardiovascular system
7. respiratory system
8. reproductive system

### 6) INDUSTRIAL APPLICATIONS OF THE INVENTION

Bioactive sequences of the pro-opiomelanocortin, as defined by the invention, may be applied subcutaneously, intramuscularly, intraperitoneally and intravenously. Topical applications include ointments, creams, gels, suppositories, vaginal suppositories, eye-drops and ear-drops.

### REFERENCES

1. L. Baranyi, W. Campbell, K. Ohishima et al., Nature Medicine 1 (1995) 894-901.
2. J. E. Blalock, Nature Medicine, 1(1995) 876-878.
3. N. Stambuk, On the Optimization of Complementary Protein Coding, in: S. Ohno, K. Aoki, M. Usui, E. Uchio (Eds.), Uveitis Today, Elsevier, Amsterdam, 1998, pp 315-318.
4. N. 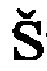 tambuk and P. Konjevoda, *Period. Biol.* **101** (1999) 363-368.
5. N. 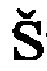 tambuk, N. Gotovac, M. Martinis et al. Simple Three-step Method for the Analysis and Design of Repetitive and Bioactive protein Motifs, in: V. B. Bajić (Ed.), Advances in Systems Signals Control and Computers vol. II, IAAMSAD, and ANS, Durban, 1998, pp 310-311.
6. P. Konjevoda, N. 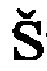 tambulc, D. Vikić-Topic et al. *Croat. Chem. Acta* **73** (2000) 1111-1121:
7. P. Konjevoda, N. 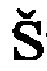 tambuk, G. Aralica and B. Pokrić, *J*. *Physiol.- Paris* **95 (1-6)**(2001) 277-281.
8. J. M. Lipton and A. Catania, Immunol, Today 18 (1997) 140-145.
9. N. 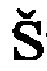 tambuk, N. Kopjar, K. 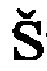 entija, et al. *Croat. Chem. Acta* **71** (1998) 591-605.

### TABLES AND FIGURES

Table 1. Combination of two bioactive anf repetitive protein motifs (peptide YGGFM, and peptide SYSMEHFRWGKPV) was determined computationally by means of the SCAN software (3-5) from the pro-opiomelanocortin precursor and pro-opiomelanocortin molecules. Repetitive peptid YGGFM is also present in the preproenkephalin, proenkephalin, lipotropin beta, endorphin beta and adrenorphin molecules (a). Peptide SYSMEHFRWGKPV is additionally present in melanotropin and corticotropin molecules (b).

Combination of bioactive peptides YGGFM and SYSMEHFR WGKPV of the pro-opiomelanocortin molecule are described herein.

Other combination of two sequences of both peptides are described herein by structural formulas:
YGGFMSYSMEHFRWGKPVYGGFM,
YGGFMSYSMEHFRWGKPV
SYSMEHFRWGKPVYGGFM.

| (a) | | |
|---|---|---|
| No. | Sequence YGGFM | Residue |
| 1 | Adrenorphin | 1-5 |
| 2 | Endorphin beta | 1-5 |
| 3 | Preproenkephalin | 54-58, 61-65, 90-94, 140-144, 164-168,215-219 |
| 4 | Proenkephalin | 100-104, 107-111, 136-140, i86-190, 210-214, 261-265 |
| 5 | Lipotropin beta | 59-63, 61-65 |
| 6 | Pro-opiomelanocortin precursor | 232-236 |
| 7 | Pro-opiomelanocortin | 3-7,237-241 |

| (b) | | |
|---|---|---|
| No. | Sequence SYSMEHFRWGKPV | Residue |
| 1 | Melanofropin | 1-13 |
| 2 | Corticotropin | 1-13 |
| 3 | Pro-opiomelanocortin precursor | 133-145 |
| 4 | Pro-opiomelanocortin | 138-150 |

Figure 1. Cytoprotective effects of the bioactive peptide fragments SYSMEHFRWGKPV and YGGFM of the pro-opiomelanocortin molekule in the rat model of the ethanol induced gastric lesions. Combination of sequences SYSNEHFRWGKPV + YGGFM exhibits stronger cytoprotective effects than individual peptide sequences.

## Claims

1. A combination of two bioactive and repetitive pro-opiomelanocortin peptide sequences for use in medicine, said sequences being SYSMEHFRWGKPV and YGGFM, wherein the combination is SYSMEHFRWGKPV and YGGFM, or is selected from YGGFMSYSMEHFRWGKPVYGGFM, YGGFMSYSMEHFRWGKPV or SYSMEHFRWGKPVYGGFM.

2. A combination of two bioactive and repetitive pro-opiomelanocortin peptide sequences according to claim 1, wherein the combination is two different peptides SYSMEHFRWGKPV and YGGFM.

3. A combination of two bioactive and repetitive pro-opiomelanocortin peptide sequences according to claim 1, wherein the peptide sequence is YGGFMSYSMEHFRWGKPVYGGFM.

4. A combination of two bioactive and repetitive pro-opiomelanocortin peptide sequences according to claim 1, wherein the peptide sequence is YGGFMSYSMEHFRWGKPV.

5. A combination of two bioactive and repetitive pro-opiomelanocortin peptide sequences according to claim 1, wherein the peptide sequence is SYSMEHFRWGKPVYGGFM.

## Patentansprüche

1. Kombination von zwei bioaktiven und repetitiven Pro-Opiomelancortin Peptidsequenzen für medizinische Zwecke, wobei die obengenannten Sequenzen SYSMEHFRWGKPV und YGGFM sind, wobei die Kombination SYSMEHFRWGKPV und YGGFM ist, oder aus YGGFMSYSMEHFRWGKPVYGGFM, YGGFMSYSMEHFRWGKPV oder SYSMEHFRWGKPVYGGFM ausgewählt ist.

2. Kombination von zwei bioaktiven und repetitiven Pro-Opiomelancortin Peptidsequenzen nach Anspruch 1, wobei es sich bei der Kombination um zwei verschiedene Peptide SYSMEHFRWGKPV und YGGFM handelt.

3. Kombination von zwei bioaktiven und repetitiven Pro-Opiomelancortin Peptidsequenzen nach Anspruch 1, wobei die Peptidsequenz YGGFMSYSMEHFRWGKPVYGGFM ist.

4. Kombination von zwei bioaktiven und repetitiven Pro-Opiomelancortin Peptidsequenzen nach Anspruch 1, wobei die Peptidsequenz YGGFMSYSMEHFRWGKPV ist.

5. Kombination von zwei bioaktiven und repetitiven Pro-Opiomelancortin Peptidsequenzen nach Anspruch 1, wobei die Peptidsequenz SYSMEHFRWGKPVYGGFM ist.

## Revendications

1. Une combinaison de deux séquences peptidiques pro-opiomélanocortines bioactives et répétitives déstinée à l'utilisation en médecine, les dites séquences étant SYSMEHFRWGKPV et YGGFM, dans laquelle la combinaison est SYSMEHFRWGKPV et YGGFM, ou est choisie parmi YGGFMSYSMEHFRWGKPVYGGFM, YGGFMSYSMEHFRWGKPV ou SYSMEHFRWGKPVYGGFM.

2. Une combinaison de deux séquences peptidiques pro-opiomélanocortines bioactives et répétitives selon la revendication 1, dans laquelle la combinaison est de deux peptides différents SYSMEHFRWGKPV et YGGFM.

3. Une combinaison de deux séquences peptidiques pro-opiomélanocortines bioactives et répétitives selon la revendication 1, dans laquelle la séquence peptidique est YGGFMSYSMEHFRWGKPVYGGFM.

4. Une combinaison de deux séquences peptidiques pro-opiomélanocortines bioactives et répétitives selon la revendication 1, dans laquelle la séquence peptidique est YGGFMSYSMEHFRWGKPV.

5. Une combinaison de deux séquences peptidiques pro-opiomélanocortines bioactives et répétitives selon la revendication 1, dans laquelle la séquence peptidique est SYSMEHFRWGKPVYGGFM.
